# EUROPEAN PATENT SPECIFICATION

(11) **EP 2 393 349 B1**
(45) Date of publication and mention of the grant of the patent: **16.10.2024**
(21) Application number: 10708946.8
(22) Date of filing: 05.02.2010
(51) Int. Cl.: A01H 6/20, C12Q 1/6895, A01H 1/04, A01H 1/00

(54) **XANTHOMONAS CAMPESTRIS PV. CAMPESTRIS RESISTANT BRASSICA PLANT AND PREPARATION THEREOF**
GEGEN XANTHOMONAS CAMPESTRIS PV. CAMPESTRIS RESISTENTE BRASSICA-PFLANZE UND DEREN HERSTELLUNG
BRASSICA RÉSISTANTE À L'INFECTION PAR XANTHOMONAS CAMPESTRIS PV. CAMPESTRIS ET SA PREPARATION

(30) Priority: 06.02.2009 NL 1036531
(43) Date of publication of application: 14.12.2011
(73) Proprietor: Bejo Zaden B.V., 1749 CZ Warmenhuizen (NL)
(72) Inventor: LIGTHART, Johannes, Theodorus, Wilhelmus, NL-1749 GZ Warmenhuizen (NL); VEENSTRA, Roelof, Marinus, NL-1766 HC Wieringerwaard (NL); BIERSTEKER, Klaas, NL-1834 TT Sint Pancras (NL); DE GEUS, Jan, NL-1724 XX Oudkarspel (NL); HUITS, Hendrikus, Stephanus, Maria, NL-1705 HP Heerhugowaard (NL); SCHRIJVER, Albertus, Johannes, Maria, NL-1749 KH Warmenhuizen (NL)
(74) Representative: van Kooij, Adriaan
(86) International application number: PCT/EP2010/051426
(87) International publication number: WO 2010/089374

(56) References cited:
- US-B2- 8 445 752
- "RAPDs-OperonandBCPrimerSequences", 1993, Retrieved from the Internet <URL:gopher://s27w007.pswfs.gov/11/.Data/.Primers/.RAPD>
- "LI-COR 4200 Series genetic analysis manual", 1997, LI-COR BIOTECHNOLOGY DIVISION
- SOENGAS P ET AL: "Identification of quantitative trait loci for resistance to Xanthomonas campestris pv. campestris in Brassica rapa", THEORETICAL AND APPLIED GENETICS ; INTERNATIONAL JOURNAL OF PLANT BREEDING RESEARCH, SPRINGER, BERLIN, DE, vol. 114, no. 4, 12 December 2006 (2006-12-12), pages 637 - 645, XP019487569, ISSN: 1432-2242
- TAYLOR J D ET AL: "Sources and origin of resistance to Xanthomonas campestris pv. campestris in Brassica genomes", PHYTOPATHOLOGY, vol. 92, no. 1, January 2002 (2002-01-01), pages 105 - 111, XP002528441, ISSN: 0031-949X
- CAMARGO L E A ET AL: "Mapping of quantitative trait loci controlling resistance of Brassica oleracea to Xanthomonas campestris pv. campestris in the field and greenhouse", PHYTOPATHOLOGY, vol. 85, no. 10, 1995, pages 1296 - 1300, XP002528442, ISSN: 0031-949X
- VICENTE J G ET AL: "Inheritance of race-specific resistance to Xanthomonas campestris pv. campestris in Brassica genomes", PHYTOPATHOLOGY, vol. 92, no. 10, October 2002 (2002-10-01), pages 1134 - 1141, XP002528443, ISSN: 0031-949X

## Description

The present invention relates to a Xanthomonas campestris pv. campestris (Xcc) resistant Brassica plant, and seeds, fruits and/or plant parts thereof.

The microorganism Xanthomonas campestris pv. campestris is the primary causative agent of blackrot in Cruciferae. Worldwide, the microorganism probably is the main pathogen for diseases of Cruciferae. Blackrot is commonly found in parts of Europe, America, Africa, Asia, Australia and Oceania. The main host plant for this bacterial disease is Brassica oleracea. However, blackrot is also found in other Cruciferae, weeds and ornamental plants.

Infection with Xanthomonas campestris pv. campestris generally occurs through hydathodes of the leaves or in some cases through stomata or injuries. Following primary infection, the microorganism spreads through the vascular bundles thereby causing black veins and V-shaped lesions in the leaves. As a consequence, a part, or parts, of the leaf withers and yellows.

Xanthomonas campestris pv. campestris is a seed transmissible disease capable of infecting plants from seeds in early stages of development. The microorganism is capable of surviving on seeds for a time period up to three years. Infection with the microorganism can also occur through plant part storage, secondary host plants and irrigation systems.

Control of the disease through chemical agents is not possible. The only measures available to combat the disease are the use of disease free, i.e., microorganism free, starting materials and sanitary measures such as removal of infected host plants. Disease free starting material can be obtained by the use of pathogen free seeds or by physically treating infected seeds. The use, and the availability, of resistant plant species, which also remain free from Xcc infection during the growth season, is strongly preferred in order to cultivate healthy plants, i.e. Xcc free plants.

Brassica is a plant genus of the family of Brassicaceae (formerly referred to as Cruciferae). The members of this genus are also known as cabbage or mustard. The genus Brassica comprises a number of important agricultural and horticultural crops, including rape, cauliflower, red cabbage, savoy cabbage, white cabbage, oxheart cabbage, curly cale cabbage, broccoli, Brussels sprouts, Chinese cabbage, turnip cabbage and Portuguese cabbage (tronchuda).

A variety of plant parts of Brassica plants is used for consumption such as roots (turnip), stalks (turnip cabbage), leaves (e.g. white and red cabbage), axillary buds (sprouts), flowers (cauliflower, broccoli). Further, rape and rape seed are also used for providing vegetable oils. Some species with white or purple flowers, or a distinct colour or shape of the leaves, are cultivated for ornamental purposes.

Considering the importance of Brassica plants for food production and the economic losses associated with Xanthomonas campestris pv. campestris infection, it is an object, amongst other objects, of the present invention to provide a Xcc resistant Brassica plant.

The need for a Xcc resistant Brassica plant is further indicated by the absence of adequate, cost-effective, and efficient means for controlling blackrot. For instance, there is no biocide available against the bacterium.

The above objects of the present invention, amongst other objects, are met the appended claims.

Specifically, above objects of the present invention, amongst other objects, are met by a *Xanthomonas campestris* pv. *campestris* resistant *Brassica* plant homozygously comprising in its genome a Quantitative Trait Locus 1 (QTL1) and a Quantitative Trait Locus 2 (QTL2) of a plant with deposit number NCIMB 41553;
said Quantitative Trait Locus 1 (QTL1) is characterized by RAMP markers consisting of a fragment of 158 to 162 bp with primer combination SEQ ID No: 1 and primer 6; a fragment of 283 to 287 bp with primer combination SEQ ID No: 2 and primer 6; a fragment of 370 to 374 bp with primer combination SEQ ID No: 3 and primer 6; and a fragment of 41 to 45 bp with primer combination SEQ ID No: 4 and primer 6; and
said Quantitative Trait Locus 2 (QTL2) is characterized by RAMP markers consisting of a fragment of 88 to 92 bp with primer combination SEQ ID No: 5 and primer 6; a fragment of 125 to 129 bp with primer combination SEQ ID No: 6 and primer 6; a fragment of 334 to 338 bp with primer combination SEQ ID No: 7 and primer 6; and a fragment of 47 to 51 bp with primer combination SEQ ID No: 8 and primer 6;
wherein primer 6 denotes Operon RAPD^{®} 10-mer kits A-01 to BH20; and
wherein said *Xanthomonas campestris* pv. *Campestris* resistant *Brassica* plant is obtained by a method comprising:
   - providing *Xanthomonas campestris* pv. *Campestris* resistant *Brassica* plant with deposit number NCIMB 41553;
   - introgressing of, or genomically combining, Quantitative Trait Locus 1 (QTL1) and Quantitative Trait Locus 2 (QTL2) from NCIMB 41553 in a *Brassica* acceptor plant using isolated genomic material of the acceptor plant and SEQ ID Nos 1 to 8 and RAPD primers from Operon RAPD 10-mer kits A-01 - BH-20 for identifying QTL 1 and QTL 2.

The Brassica acceptor plant according to the present invention is preferably a Brassica oleracea plant, preferably chosen from the group consisting of Brassica oleracea convar. botrytis var. botrytis (cauliflower, romanesco), Brassica oleracea convar. botrytis var. cymosa (broccoli), Brassica oleracea convar. botrytis var. asparagoides (sprouting broccoli), Brassica oleracea convar. oleracea var. gemnifera (Brussels sprouts), Brassica oleracea convar. capitata var. alba (white cabbage, oxheart cabbage), Brassica oleracea convar. capitata var. rubra (red cabbage), Brassica oleracea convar. capitata var. sabauda (savoy cabbage), Brassica oleracea convar. acephela var. sabellica (curly cale cabbage), Brassica oleracea convar. acephela var. gongyloides (turnip cabbage) and Brassica oleracea var. tronchuda syn. costata (Portuguese cabbage).

Considering the advantages of a Xcc resistant Brassica plant described above, the present invention relates, according to a further aspect, to seeds, fruits and/or other plant parts thereof, comprising in its genome a Quantitative Trait Locus 1 (QTL1) and a Quantitative Trait Locus 2 (QTL2) as defined above.

The present invention will be further exemplified using the following example of a preferred embodiment. This example is merely intended as illustrative, and not to limit the scope of the present invention as outlined in the appended claims by any means.

### Brief description of the drawings

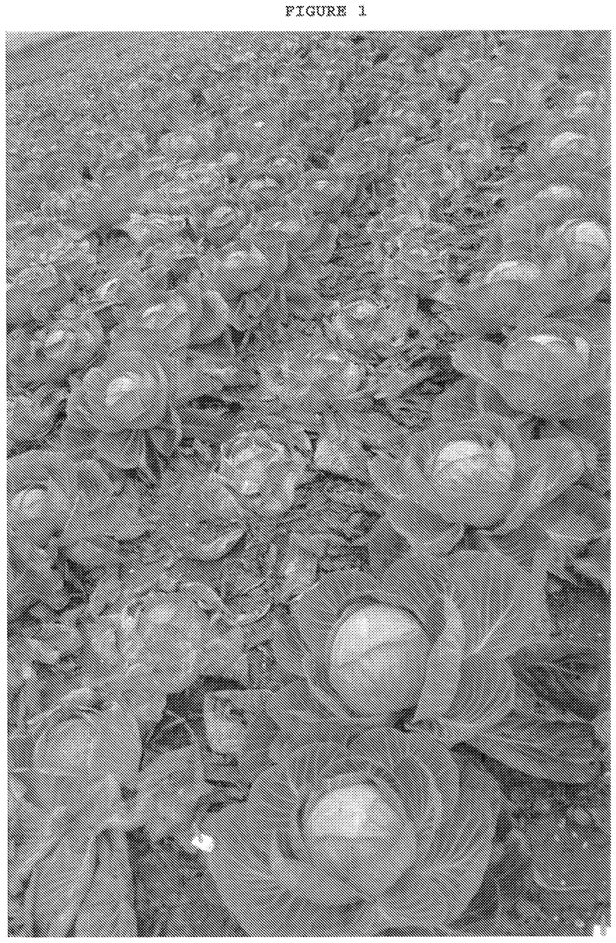

- **Figure 1**: shows a field test wherein Xcc resistant and Xcc susceptible Brassica's are present.

### Example

The breeding of Xanthomonas campestris pv. campestris (Xcc) resistance into Brassica oleracea is a complex process. Several varieties of Xcc are described and Xcc resistance is generally not observed in Brassica oleracea. The presently described resistances of Brassica oleracea to Xcc are of quantitative nature, i.e. the resistance, and the level thereof, is dependent on the genetic background. This implies that several genes are involved in this resistance, resulting in that degrees of resistance to Xcc may be observed.

In order to obtain a qualitative resistant Brassica oleracea plant, i.e., the resistance is observed independent of the genetic background, a genetic source is used in which the resistance is essentially recessive. Through a program of backcrossing, the resistance is introduced in various genetic backgrounds (susceptible parent lines). The resistance level of the plants is determined by a field test. Since the trait involved is recessive, each crossing with a susceptible plant is followed by an inbreed generation in order to obtain the trait in a homozygous form. The progeny of this generation must be tested for their resistance level in a field test. As a result, it will take at least two years for an annual and at least four years for a bi-annual plant, in order to visualize an effect of the new crossing.

Since the resistance involved can be expressed as different levels of resistance, the resistance is expressed in quantitative values (on a scale from 0 to 9, where 0 represents fully susceptibility and 9 represents completely resistance). It was shown that the resistance in the various genetic backgrounds showed a phenotypic gradation within a normal distribution, instead of a simple Mendelian inheritance. This distribution can shift towards susceptible implying that the inbreed generations, from crossings between the resistant source and various susceptible parent lines, provide different levels of resistant plants.

Based on the segregation ratios and the differences in resistance levels, it was demonstrated that the resistance level of the plant is determined by several genetic factors or Qualitative Traits Loci. In order to identify DNA markers indicative, or representative, of a quantitative trait, QTL analyses are often used. QTL's are independent chromosomal regions which, coupled to underlying genes, in combination explain, or indicate, a genetic trait.

Using DNA markers, a genome spanning QTL analysis was performed on Brassica oleracea populations with diverse genetic backgrounds. With plants of these diverse populations, field tests were performed in order to determine the resistance level of the individual plants. The data of the marker analysis and the scores of the field test enabled identification of QTL's responsible for the Xcc resistance observed.

In total, six independently inherited QTL's were identified contributing, in some degree, to the resistance level observed. The variation of resistance levels observed in the field between the plants is a result of the presence and/or absence of the QTL's and the various combinations thereof. Of the six QTL's identified, two QTL's, designated herein as QTL 1 and QTL 2, were present in each genetic background providing resitance. In other words, these two QTL's are indicative of a qualitative resistance while the other four remainder of the QTL's appear to contribute to quantitative resistance. One of these QTL's, i.e. QTL 1, can be considered as the major-QTL, i.e., the QTL providing the strongest contribution to the resistance observed.

Each Xanthomonas campestris pv. campestris resistant inbreed line obtained by the breeding process possesses this major-QTL. The second QTL has, similar to the major-QTL, a contribution to the quantitative resistance level. However, this contribution is not as large as from the major-QTL, although it is also independent of the genetic background.

The remaining four QTL's are found in the different genetic backgrounds. These four QTL's may have a modifying role which results in variation of the resistance level.

This is exemplified in table 1 below, where Brassica oleracea plants 1 to 6 are examples to illustrate these observations.

**Table 1 Overview of the influence of the several QTL's on the resistance to Xcc.**

| | QTL1 | QTL2 | QTL3 | QTL4 | QTL5 | QTL6 | Level of resistance |
|---|---|---|---|---|---|---|---|
| plant 1 | + | + | + | + | + | + | resistant |
| plant 2 | + | + | + | + | + | + | resistant |
| plant 3 | - | - | - | - | - | - | susceptible |
| plant 4 | - | + | + | + | + | + | susceptible |
| plant 5 | + | - | + | + | + | + | susceptible |
| plant 6 | - | - | + | + | + | + | susceptible |

| | | | | | | | |
|---|---|---|---|---|---|---|---|
| Legend to this table: + present ± may be present, depending on the exact genetic background. - absent | | | | | | | |

The use of DNA markers associated with the two major QTL's, i.e. QTL 1 and QTL 2, provided the opportunity to select for plants being resistant to Xcc.

A disease test alone will not inherently provide plants having both QTL's in a homozygous form. The disease test may also result in plants with a high resistance level having one or more QTL's in a heterozygous form which inherently will not provide a stabile resistance.

Performing a disease test with more than thousand plants is only possible for a limited number of crossings. However, use of DNA markers offered the opportunity to analyse more populations consisting of one thousand plants in order to preselect desired plants.

Besides this, the selection using DNA markers also offered the opportunity to perform repeated backcrossing wherein the two major QTL's were maintained. This method gained one or two year per crossing generation which means acceleration in the breeding program of ten till twenty years. This quicker method provides an accelerated introduction of the novel Xcc resistance into hybrid varieties.

An additional complicating factor in the production of hybrid varieties is the required presence of the recessive resistance in both parents. Preferably, both parental lines, or donor plants, are homozygous for the two major QTL's. By using DNA markers, the identification of parents homozygous for the two major QTL's and, accordingly, suitable for producing a new resistant hybrid, can be performed in a relatively short period of time.

Seeds of such a hybrid are deposited at the NCIMB, Aberdeen, Scotland, AB21, 9YA, UK with deposit number NCIMB 41553.

The two major QTL's are both characterized by four DNA markers which characterize the presence thereof in the source. QTL1 is the major QTL, and QTL2 is the minor QTL.

The DNA markers were generated by the RAMP technique. The RAMP technique, wherein an iSSR and a RAPD-primer are combined, provides band patterns comprising one or more DNA fragments therein specifically co-segregating with the resistance. By mapping the R_AMP-fragments and phenotype resistance scores, closely linked RAMP-markers were identified which identify the QTL **(table 2).** The genetic distance between the DNA markers within the QTL is represented by centimorgans (cM).

The general PCR conditions used for generating the DNA markers were as follows:

### PCR mix for RAMP reaction:

### Per reaction

~ 0.2 ng/µl genomic plant DNA
75 mM Tris-HCL (pH 8.8)
20 mM NH₄SO₄
0.01% (v/v) Tween20
2.8 mM MgCl₂
0.25 mM dNTPs
0.15 µM forward primer
0.20 µM reverse primer
0.04 units/µl Red Hot^{®} DNA Polymerase (ABgene, Epsom)

### PCR program RAPD35:

| | | cycles |
|---|---|---|
| step 1: | 2 min. 93°C | 1 |
| step 2: | 30 sec. 93°C | |
| step 3: | 30 sec. 35°C | |
| step 4: | heating with 0.3°/sec to 72°C | |
| step 5: | 1 min. 30 sec 72°C repeat steps 2-5 | 40 |
| step 6: | 5 min 72°C | 1 |

### PAGE/Licor:

For analysis of the RAMP patterns use was made of a "Gene ReadIR 4200 DNA analyzers" (Licor Inc.). On the basis of an optimal concentration of 6.5% acryl amide, fragments can be separated which have a difference in size of a single base.

In order to visualise the fragments in this system, it is necessary to use labelled (IRDye labels) primers. For this purpose a one/third of the amount of forward primer was replaced by a labelled primer with identical sequence.

### Marker overview

In the research leading to the present invention, the primers as shown in **table 3** were used for generating the DNA markers as shown in **table 2.**

**Table 2: Overview of RAMP markers per QTL**

| QTL | RAMP primer combination | Fragment size (bp) | Position in QTL (cM) |
|---|---|---|---|
| 1 | 1.1 + 6 | 160 | 10,1 |
| 1 | 1.2 + 6 | 285 | 18,5 |
| 1 | 1.3 + 6 | 372 | 20,9 |
| 1 | 1.4 + 6 | 43 | 21,3 |
| 2 | 2.1 + 6 | 90 | 28,5 |
| 2 | 2.2 + 6 | 127 | 29, 4 |
| 2 | 2.3 + 6 | 336 | 36, 3 |
| 2 | 2.4 + 6 | 49 | 37,2 |

**Table 3: Overview of SEQ ID Nos**

| SEQ ID No. | Primer | iSSR/RAPD | Sequence |
|---|---|---|---|
| 1 | 1.1 | iSSR | TTA GCT CTC TCT CTC TC |
| 2 | 1.2 | iSSR | CCA GCA CAC ACA CAC A |
| 3 | 1.3 | iSSR | AGA TTC TCT CTC TCT C |
| 4 | 1.4 | iSSR | CAA CTC TCT CTC TCT |
| 5 | 2.1 | iSSR | TTG TAG AGA GAG AGA G |
| 6 | 2.2 | iSSR | TCT CTT CTT CTT CTT C |
| 7 | 2.3 | iSSR | CAA CTC TCT CTC TCT |
| 8 | 2.4 | iSSR | GAA ATC TCT CTC TCT C |
| | | | |
| | 6 | RAPD | Operon RAPD^{®} 10-mer kits A-01 to BH20 (Operon Biotechnologies, Inc., Huntsville, USA |

The PCR reactions with the various primer combinations provide nucleic acid fragments with a basepair size indicated (see **table 2**) representative for the presence of the respective QTL. These DNA-markers are characteristic, or indicative, for the QTL's concerned. The combination of these DNA-markers, characterising the QTL, provides indisputable evidence of the presence of the QTL introgression from the Xcc resistant source in the donor plant.

### Definitions

**cM** - **centimorgan** - Unit for the genetic distance between markers, based on the number of crossovers per hundred individuals.

**DNA marker** - A DNA fragment which is linked to a gene or another DNA fragment with a known location on the genome, which is used to monitor the inheritance of this gene or this location.

**Gel-electrophoresis** - Method for separating molecules (DNA, RNA, proteins), on the basis of their size, shape or charge, in a matrix (agarose or polyacrylamide) under the influence of an electric field.

**Inbred generation (self pollination)** - Fertilization of an individual with its own pollen.

**Introgression** - A chromosome fragment of a line (cultivar) introduced, by way of crossing, into another line (cultivar).

**IRDye labels** - Infrared labels used for Licor imaging systems, the detection of which takes place at 700 nm or 800 nm.

**Monogenic** - Determined by a single gene.

**PCR** (Polymerase Chain Reaction) - An *in vitro* amplification method for multiplying a specific DNA fragment. This synthesis reaction makes use of a minimum of one oligonucleotide primer which hybridizes with a DNA fragment, after which a DNA polymerase amplifies the flanking region via successive temperature cycles.

**Primer** - A short oligonucleotide (-20-50bp) complementary to the sequence of a single-strand DNA molecule, which serves as starting point of a polymerase.

**QTL** (Quantitative Trait Locus) - an independent chromosome region(s) which, when coupled to genes, together explain a trait.

**RAMPs** (Random Amplified Microsatellite Polymorphisms) - DNA fingerprinting technique based on RAPD and iSSR primers with which polymorphisms between different DNA samples are detected.

**RAPD-primer** (Random Amplified Polymorphic DNA primer) - A 10-mer with a "random" sequence, wherein the GC-content lies between 60% and 70% and wherein the primer ends are not self-complementary.

**iSSR** (inter Simple Sequence Repeat) - A primer designed on the 5' end of an SSR (Single Sequence Repeat); a DNA fragment consisting of repetitions of 2 or 3 nucleotides

**BC** (Backcrossing) - Crossing of an individual with one of the original parents.

**XCC** Xanthomonas campestris pv. campestris.

## Claims

1. *Xanthomonas campestris* pv. *campestris* resistant *Brassica* plant homozygously comprising in its genome a Quantitative Trait Locus 1 (QTL1) and a Quantitative Trait Locus 2 (QTL2) of a plant with deposit number NCIMB 41553;
said Quantitative Trait Locus 1 (QTL1) is **characterized by** RAMP markers consisting of a fragment of 158 to 162 bp with primer combination SEQ ID No: 1 and primer 6; a fragment of 283 to 287 bp with primer combination SEQ ID No: 2 and primer 6; a fragment of 370 to 374 bp with primer combination SEQ ID No: 3 and primer 6; and a fragment of 41 to 45 bp with primer combination SEQ ID No: 4 and primer 6; and
said Quantitative Trait Locus 2 (QTL2) is **characterized by** RAMP markers consisting of a fragment of 88 to 92 bp with primer combination SEQ ID No: 5 and primer 6; a fragment of 125 to 129 bp with primer combination SEQ ID No: 6 and primer 6; a fragment of 334 to 338 bp with primer combination SEQ ID No: 7 and primer 6; and a fragment of 47 to 51 bp with primer combination SEQ ID No: 8 and primer 6;
wherein primer 6 denotes Operon RAPD^{®} 10-mer kits A-01 to BH20; and
wherein said *Xanthomonas campestris* pv. *Campestris* resistant *Brassica* plant is obtained by a method comprising:
- providing *Xanthomonas campestris* pv. *Campestris* resistant *Brassica* plant with deposit number NCIMB 41553;
- introgressing of, or genomically combining, Quantitative Trait Locus 1 (QTL1) and Quantitative Trait Locus 2 (QTL2) from NCIMB 41553 in a *Brassica* acceptor plant using isolated genomic material of the acceptor plant and SEQ ID Nos 1 to 8 and RAPD primers from Operon RAPD 10-mer kits A-01 - BH-20 for identifying QTL 1 and QTL 2.

2. Seeds, fruits and/or other plant parts of a *Brassica* plant according to claim 1 which seeds, fruits and/or other plant parts comprise said Quantitative Trait Locus 1 (QTL1) and said Quantitative Trait Locus 2 (QTL2).

## Patentansprüche

1. Xanthomonas campestris pv. campestris-resistente Brassica-Pflanze, die homozygot in ihrem Genom einen Quantitative Trait Locus 1 (QTL1) und einen Quantitative Trait Locus 2 (QTL2) einer Pflanze mit der Depotnummer NCIMB 41553 umfasst;
wobei der Quantitative Trait Locus 1 (QTL1) **durch** RAMP-Marker **gekennzeichnet** ist, bestehend aus einem Fragment von 158 bis 162 bp mit Primer-Kombination SEQ ID Nr.: 1 und Primer 6; einem Fragment von 283 bis 287 bp mit Primer-Kombination SEQ ID Nr.: 2 und Primer 6; einem Fragment von 370 bis 374 bp mit Primer-Kombination SEQ ID Nr.: 3 und Primer 6; und einem Fragment von 41 bis 45 bp mit Primer-Kombination SEQ ID Nr.: 4 und Primer 6; und
wobei der Quantitative Trait Locus 2 (QTL2) **durch** RAMP-Marker **gekennzeichnet** ist, bestehend aus einem Fragment von 88 bis 92 bp mit Primer-Kombination SEQ ID Nr.: 5 und Primer 6; einem Fragment von 125 bis 129 bp mit Primer-Kombination SEQ ID Nr.: 6 und Primer 6; einem Fragment von 334 bis 338 bp mit Primer-Kombination SEQ ID Nr.: 7 und Primer 6; und einem Fragment von 47 bis 51 bp mit Primer-Kombination SEQ ID Nr.: 8 und Primer 6;
wobei Primer 6 die Operon RAPD^{®} 10-Mer-Kits A-01 bis BH20 bezeichnet; und
wobei die Xanthomonas campestris pv. Campestris-resistente Brassica-Pflanze durch ein Verfahren gewonnen wird, umfassend:
- Bereitstellen einer Xanthomonas campestris pv. Campestris-resistenten Brassica-Pflanze mit der Depotnummer NCIMB 41553;
- Introgression von oder genomische Kombination von Quantitative Trait Locus 1 (QTL1) und Quantitative Trait Locus 2 (QTL2) aus NCIMB 41553 in einer Brassica-Akzeptorpflanze unter Verwendung von isoliertem genomischem Material der Akzeptorpflanze und den SEQ ID Nr. 1 bis 8 und RAPD-Primern aus den Operon RAPD 10-mer Kits A-01 - BH-20 zur Identifizierung von QTL 1 und QTL 2.

2. Samen, Früchte und/oder andere Pflanzenteile einer Brassica-Pflanze nach Anspruch 1, deren Samen, Früchte und/oder andere Pflanzenteile den Quantitative Trait Locus 1 (QTL1) und den Quantitative Trait Locus 2 (QTL2) umfassen.

## Revendications

1. Plante du genre *Brassica* résistante à la *Xanthomonas campestris* pv. *campestris* comprenant de manière homozygote dans son génome un locus à caractère quantitatif 1 (QTL1) et un locus à caractère quantitatif 2 (QTL2) d'une plante portant le numéro de dépôt NCIMB 41553 ;
ledit locus à caractère quantitatif 1 (QTL1) est **caractérisé par** des marqueurs RAMP consistant en un fragment allant de 158 à 162 pb avec une combinaison d'amorces SEQ ID No: 1 et une amorce 6 ; un fragment allant de 283 à 287 pb avec une combinaison d'amorces SEQ ID No: 2 et une amorce 6 ; un fragment allant de 370 à 374 pb avec une combinaison d'amorces SEQ ID No: 3 et une amorce 6 ; et un fragment allant de 41 à 45 pb avec une combinaison d'amorces SEQ ID No: 4 et une amorce 6 ; et
ledit locus à caractère quantitatif 2 (QTL2) est **caractérisé par** des marqueurs RAMP consistant en un fragment allant de 88 à 92 pb avec une combinaison d'amorces SEQ ID No: 5 et une amorce 6 ; un fragment allant de 125 à 129 pb avec une combinaison d'amorces SEQ ID No: 6 et une amorce 6 ; un fragment allant de 334 à 338 pb avec une combinaison d'amorces SEQ ID No: 7 et une amorce 6 ; et un fragment allant de 47 à 51 pb avec une combinaison d'amorces SEQ ID No: 8 et une amorce 6 ;
dans laquelle l'amorce 6 désigne des kits Operon RAPD^{®} 10-mer A-01 à BH20 ; et
dans laquelle ladite plante du genre *Brassica* résistante à la *Xanthomonas campestris* pv. *Campestris* est obtenue par un procédé comprenant :
- la fourniture de la plante du genre *Brassica* résistante à la *Xanthomonas campestris* pv. *Campestris* portant le numéro de dépôt NCIMB 41553 ;
- l'introgression ou la combinaison génomique du locus à caractère quantitatif 1 (QTL1) et du locus à caractère quantitatif 2 (QTL2) provenant de NCIMB 41553 dans une plante réceptrice du genre *Brassica* à l'aide d'un matériel génomique isolé de la plante réceptrice et des SEQ ID No 1 à 8 et des amorces RAPD provenant des kits Operon RAPD 10-mer A-01 à BH-20 pour identifier le QTL 1 et le QTL 2.

2. Graines, fruits et/ou autres parties de plantes d'une plante du genre *Brassica* selon la revendication 1, lesquels graines, fruits et/ou autres parties de plantes comprennent ledit locus à caractère quantitatif 1 (QTL1) et ledit locus à caractère quantitatif 2 (QTL2).
